Europäisches Patentamt

European Patent Office

Office européen des brevets

(11) Numéro de publication: **0 331 637**
**A1**

## (12) DEMANDE DE BREVET EUROPEEN

(21) Numéro de dépôt: **89810143.1**

(22) Date de dépôt: **22.02.89**

(51) Int. Cl.4: **A 61 N 1/16**

(30) Priorité: **22.02.88 CH 645/88**

(43) Date de publication de la demande:
**06.09.89 Bulletin 89/36**

(84) Etats contractants désignés:
**AT BE DE ES FR GB GR IT LU NL SE**

(71) Demandeur: **PROGAM S.A.**
**La Lechere, c/o Denis Guignard**
**CH-1141 Yens (CH)**

(72) Inventeur: **Curchod, Jacques**
**Beaulieu 27**
**CH-1004 Lausanne (CH)**

(74) Mandataire: **Charbonnier, Georges R.**
**8, Avenue Peschier**
**CH-1206 Genève (CH)**

(54) Emetteur d'ondes de forme.

(57) L'émetteur d'ondes de forme est constitué par un canon (10) composé de quatre éléments coniques coaxiaux (11,12,13,14), en matériau conducteur d'électricité, formant un ensemble d'un seul tenant.

Ce canon (10) présente deux points de convergence (S) constitués par les sommets des cônes terminaux (11,14).

Fig. 1

Bundesdruckerei Berlin

EP 0 331 637 A1

**Description**

## EMETTEUR D'ONDES DE FORME

Selon une théorie biologique généralement admise par les hommes de science, toute structure émet, en fonction de sa forme, de sa nature, de son volume, des ondes électro-magnétiques dites de forme.

Si les intensités de ces ondes sont extrêmement faibles, si leurs longueurs et les énergies qu'elles véhiculent sont aujourd'hui encore difficiles à chiffrer, en revanche leurs effets sont tout à fair perceptibles sur les êtres humains et leur environnement.

Les ondes de forme ont des propriétés diverses positives ou négatives, c'est-à-dire bénéfiques ou nocives. Elles se manifestent de manière particulièrement nocive lorsqu'elles détruisent, dans un espace déterminé, l'équilibre des forces cosmo-telluriques.

Pour réduire, sinon anhiler les effets néfastes de telles ondes, on n' avait pas d'autre ressource jusqu'à présent que celle d'éloigner les objets et les êtres vivants des sources responsables de ces radiations. Malheureusement l'homme moderne étant souvent intégré à un milieu hostile de ce point de vue , on prendra pour seul exemple celui des personnes travaillant avec des ordinateurs, cette solution est souvent inapplicable.

La présente invention apporte une nouvelle solution à ce problème en permettant littéralement de corriger, dans le sens d'une amélioration, les conditions de vie, de travail, des personnes exposées à des ondes de forme nocives.

Elle a pour objet un émetteur d'ondes de forme qui permet de compenser, dans un espace donné, les ondes de forme qui engendrent un déséquilibre de l'harmonie des forces cosmo-telluriques.

Cet émetteur est caractérisé par le fait qu'il comprend au moins un canon générateur d'ondes de forme, composé d'une suite de plusieurs éléments coaxiaux, conducteur d'électricité ou non, de formes géométriques analogues ou identiques, reliés entre eux de manière à constituer un ensemble rigide se terminant , au moins à l'une de ses extrémités, par un point de convergence, réel ou virtuel, situé sur l'axe du canon.

Les ondes de forme émises par les émetteurs répondant à cette définition sont engendrés par les particules élémentaires, en particulier les électrons libres et éventuellement des particules abstraites, en mouvement dans la matière. Elles se propagent, en vertu du pouvoir des pointes, principalement dans l'axe du canon.

L'émetteur peut s'étandre dans un space à deux dimensions, c'est-à-dire dans un plan, ou dans un espace à trois dimensions, c'est-à-dire qui les éléments composant le ou les canons occuperont un certain volume. Pour obtenir le rendement le plus favorable, les angles caractéristiques des éléments ne devraient pas être inférieurs à 12,5 ° ni supérieurs à 153°.

Le dessin ci-annexé représente, schématiquement et à titre d'exemple, plusieurs formes d'exécution de l'objet de l'invention.

Les figures 1 à 4 sont respectivement deux vues en élévation et deux vues verticales en perspectives de quatre formes d'exécution comprenant chacune un seul canon.

Les figures 5 et 6 sont des vues en élévation, respectivement de face et de profil, d'une cinquième forme d'exécution comprenant quatre canons.

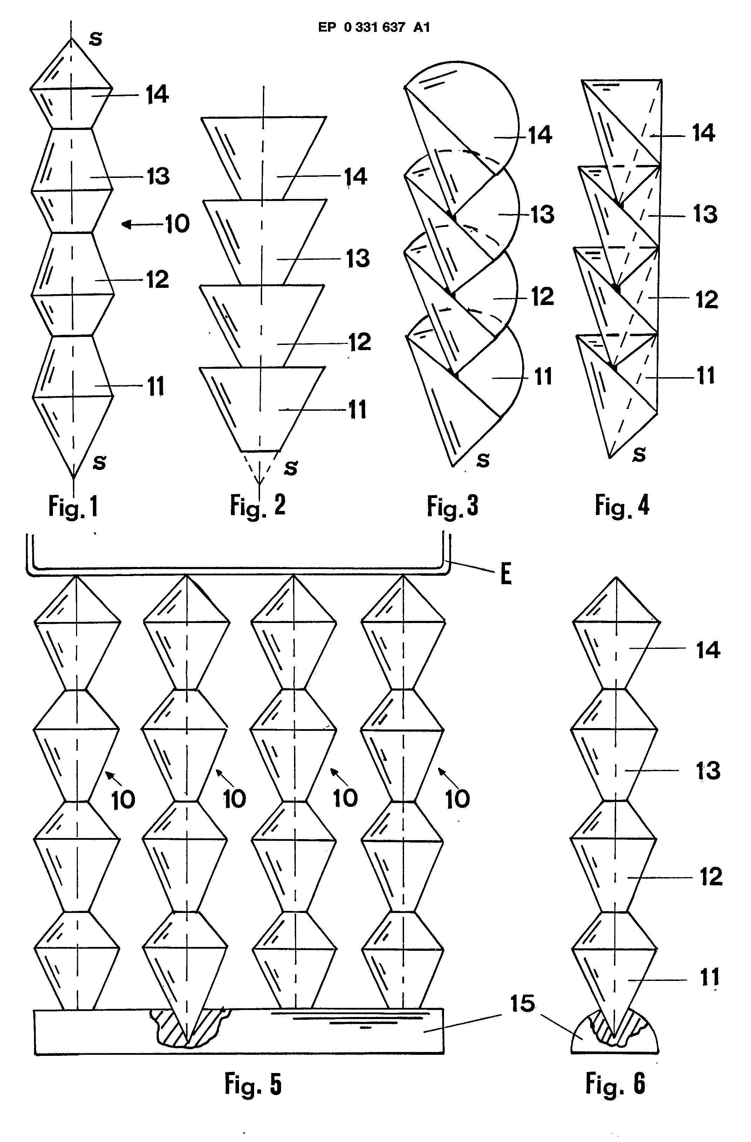

L'émetteur représenté à la figure 1 comprend un canon 10 composé de quatre éléments métalliques 11, 12, 13 et 14 présentant individuellement la forme de double cône. Les cônes intermédiaires sont tronqués contrairement aux cônes terminaux.

Les quatre éléments 11, 12, 13 et 14 constituent un ensemble d'un seul tenant conducteur d'électricité qui peut être réalisé par exemple par décolletage d'un cylindre.

Ce canon comprend deux points de convergence (S) réels constitués par les pointes des éléments terminaux.

L'émetteur représenté à la figure 2 comprend un canon 10 composé de quatre éléments métalliques 11, 12, 13 et 14 constitués individuellement par des cônes tronqués identiques.

Ces quatre éléments 11, 12, 13 et 14 forment, comme dans la première forme d'exécution, un ensemble d'une seule pièce également réalisable par décolletage.

Ce canon comprend un point de convergence virtuel (S) constitué par le sommet fictif de l'élément tronconique 11.

L'émetteur représenté à la figure 3 comprend un canon 10 composé de quatre éléments métalliques identiques, 11, 12, 13 et 14 présentant la forme d'un demi-cône.

Ces demi-cône sont fixés l'un à l'autre de manière à constituer un ensemble rigide conducteur de l'électricité.

Cet ensemble pourra être réalisé soit à partir d'élément demi-coniques individuels fixés l'un à l'autre par un point de soudure entre le sommet du demi-cône de l'un et l'arête diamétrale de la demi-base de l'autre, ou en coupant axialement un ensemble de quatre éléments coniques reliés entre eux par un pont de matière au niveau de leurs sommets.

L'émetteur représenté à la figure 4 comprend au canon 10 composé de quatre éléments métalliques identiques 11, 12, 13 et 14 présentant la forme d'un polyèdre à quatre faces.

Ces éléments, sont reliés entre eux comme les demi-cônes de la troisième forme d'exécution, de manière à constituer un ensemble d'un seul tenant conducteur de l'électricité.

Dans ces deux dernières formes d'exécution, les points de convergence, (S) à partir desquels se produits l'émission des ondes de forme, sont réels et constituer par les sommets des demi-cônes 11, respectivement des polyèdres 11.

Dans les quatre formes d'exécution représentées aux figures 1 à 4 on pourra prévoir, selon les conditions de fonctionnement , soit un dispositif de mise à terre, soit des moyens pour isoler électriquement le canon et le porter à un certain potentiel.

L'émetteur représenté aux figures 5 et 6 est plus spécialement destiné à neutraliser les ondes de formes nocives émises par les écrans de TV, d'ordinateurs, etc.

Il est constitué par une batterie de quatre canons 10, du type de celui représenté à la figure 1, montés verticalement sur un support métallique 15, doté d'une borne de mise à terre.

Le support 15 est placé sous l'écran E dont les radiations sont à neutraliser de manière que les pointes des éléments 14 affleurent son bord inférieur.

En variante, on pourrait réaliser un canon avec des éléments non-conducteurs d'électricité en utilisant les propriétés des charges statiques.

**Revendications**

1. Emetteur d'ondes de forme, caractérisé par le fait qu'il comprend au moins un canon (10) générateur d'ondes de forme, composé de plusieurs éléments coaxiaux (11, 12, 13, 14) conducteurs d'électricité ou non, et reliés entre eux de manière à constituer un ensemble régide se terminant, au moins à l'une de ses extrémités, par un point de convergence (S), réel ou virtuel, situé sur l'axe du canon.

2. Emetteur selon la revendication 1, caractérisé par le fait que les angles caractéristiques des dits éléments (11, 12, 13, 14) ne sont pas inférieurs à 12,5°, ni supérieurs à 153°.

3. Emetteur selon la revendication 1, caractérisé par le fait que lesdits éléments (11, 12, 13, 14) sont coniques ou tronconiques.

4. Emetteur selon la revendication 1, caractérisé par le fait que lesdits éléments (11, 12, 13, 14) sont de forme polyèdrique.

5. Emetteur selon la revendication 1, caractérisé par le fait que ledit canon (10) est composé de quatre éléments (11, 12, 13, 14).

6. Emetteur selon la revendication 1, caractérisé par le fait qu'il comprend plusieurs canons (10) montés en batterie sur un support (15).

7. Emetteur selon la revendication 6, caractérisé par le fait que ledit support (15) est agencé de manière à permettre la mise sous tension ou la mise à terre des canons (10).

8. Emetteur selon la revendication 1, caractérisé par le fait que ledit point de convergence (S) est réel.

9. Emetteur selon la revendication 1, caractérisé par le fait que ledit point de convergence (S) est virtuel.

10. Emetteur selon la revendication 1, caractérisé par le fait que lesdits éléments (11, 12, 13, 14) s'étendent dans un espace à deux dimensions.

11. Emetteur selon la revendication 1, caractérisé par le fait que lesdits éléments (11, 12, 13, 14) s'étendent dans un espace à trois dimensions.

S

14

13

10

12

11

S

**Fig. 1**

14

13

12

11

S

**Fig. 2**

14

13

12

11

S

**Fig. 3**

14

13

12

11

S

**Fig. 4**

E

10   10   10   10

15

**Fig. 5**

14

13

12

11

15

**Fig. 6**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A- 816 132 (CHAUMERY)<br>* Page 2, ligne 89 - page 3, ligne 27; figure 3 * | 1-5,8,9 ,11 | A 61 N   1/16 |
| A | FR-A-2 329 089 (DE LA FOYE)<br>* En entier * | 1,10 | |

DOMAINES TECHNIQUES
RECHERCHES (Int. Cl.4)

A 61 N

Le présent rapport a été établi pour toutes les revendications

| Lieu de la recherche | Date d'achèvement de la recherche | Examinateur |
|---|---|---|
| LA HAYE | 18-05-1989 | LEMERCIER D.L.L. |